(19) Europäisches Patentamt
European Patent Office
Office européen des brevets

(11) Veröffentlichungsnummer: **0 054 805**
**B1**

(12) # EUROPÄISCHE PATENTSCHRIFT

(45) Veröffentlichungstag der Patentschrift: **03.07.85**

(21) Anmeldenummer: **81110176.5**

(22) Anmeldetag: **05.12.81**

(51) Int. Cl.⁴: **C 07 C 1/20,** C 07 C 11/09, C 07 C 11/10, C 07 C 31/04, C 07 C 31/08, C 07 C 29/00 // B01J29/18

(54) **Verfahren zur Herstellung von tertiären Olefinen.**

(30) Priorität: **19.12.80 DE 3048084**

(43) Veröffentlichungstag der Anmeldung: **30.06.82 Patentblatt 82/26**

(45) Bekanntmachung des Hinweises auf die Patenterteilung: **03.07.85 Patentblatt 85/27**

(84) Benannte Vertragsstaaten: **BE DE FR NL**

(56) Entgegenhaltungen:
**DE - A - 2 534 544**
**DE - B - 2 802 199**
**US - A - 4 025 575**

(73) Patentinhaber: **EC ERDÖLCHEMIE GMBH, Postfach 75 20 02, D-5000 Köln 71 (DE)**

(72) Erfinder: **Herwig, Jens, Dr., Auf dem Hügel 23, D-5000 Köln 41 (DE)**
Erfinder: **Schleppinghoff, Bernard, Dr., Adolf-Kolping-Strasse 5, D-4047 Dormagen 1 (DE)**
Erfinder: **Scheef, Hans-Volker, Goethestrasse 69, D-4047 Dormagen 1 (DE)**

(74) Vertreter: **Mann, Volker, Dr. et al, c/o Bayer Aktiengesellschaft Zentralbereich Patente Marken und Lizenzen, D-5090 Leverkusen-Bayerwerk (DE)**

**EP 0 054 805 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von tertiären Olefinen durch katalytische Spaltung ihrer n-Alkylether.

Tertiärte Olefine sind wichtige Vorprodukte zur Herstellung von Polymeren und höherwertigen Chemikalien. So erlaubt die interessante Funktion der Doppelbindung am tertiären Kohlenstoffatom die Erzeugung zahlreicher organischer Zwischenprodukte, wie beispielsweise Pinakolin und Neocarbonsäuren und durch Dehydrierung konjugierte Diolefine, wie beispielsweise Isopren, die im Kunststoff-, Pharma-, Pflanzenschutz- und Schmierstoffsektor weiterverarbeitet werden können. Die Voraussetzung für derartige Umsetzungen liegt in der Verfügbarkeit solcher tertiärer Olefine in möglichst hoher Reinheit.

Da diese tertiären Olefine hauptsächlich im Gemisch mit anderen Kohlenwasserstoffen im Produktspektrum von thermischen und katalytischen Crackern anfallen, muss zu ihrer Reindarstellung ein Isolierungsverfahren angewendet werden. Zur Isolierung der tertiären Olefine aus den genannten Kohlenwasserstoffströmen diente bisher in erster Linie das Schwefelsäure-Verfahren, bei dem zunächst die Schwefelsäureester der den tertiären Olefinen entsprechenden tertiären Alkohole gebildet wurden, die anschliessend durch Abspaltung der Schwefelsäure wieder zu den tertiären Olefinen zurückgebildet wurden. Die Probleme dieses Verfahrens sind durch die unvermeidliche Korrosion und durch die Notwendigkeit zur Aufkonzentration der verbrauchten Säure vor ihrer Rückführung gegeben. In neuerer Zeit sind Verfahren entwickelt worden, die, ausgehend von einer selektiven Veretherung der tertiären Olefine mit Alkoholen, die Reingewinnung der tertiären Olefine über die Zersetzung dieser Ether erreichen (EP-Anmeldung 0 003 305; US 3 170 000; DE-OS 2 924 869). Bei der Spaltung der tertiären Ether tritt mit steigender Reaktionstemperatur als unerwünschte Nebenreaktion in zunehmendem Masse die Bildung der Dialkylether aus den abgespaltenen n-Alkanolen auf. Ein besonderer Nachteil dieser Etherbildung ist durch das dabei entstehende Wasser gegeben, das bei einem Alkanolkreislauf vor der Veretherungsstufe wieder entfernt werden muss. Es sind daher Katalysatoren für die Etherzersetzung gesucht, deren Aktivität gross genug ist, um bei niedrigen Temperaturen zu arbeiten oder deren Selektivität hoch genug ist, um eine möglichst geringe Etherbildung zu verursachen.

Ein solcher Katalysator ist in Form von kristalliner Kieselsäure in der DE-OS 2 924 869 beschrieben. Zur Erzielung möglichst grosser Umsätze ist jedoch auch mit diesem Katalysator das Arbeiten bei den relativ hohen Temperaturen von über 190°C bis in den Bereich von etwa 350°C erforderlich.

Es wurde nun ein Verfahren zur Herstellung von tertiären Olefinen durch katalytische Spaltung ihrer n-Alkylether in Gegenwart saurer Katalysatoren bei einer Temperatur von 100–300°C und gleichzeitiger Wiedergewinnung der entsprechenden n-Alkanole gefunden, das dadurch gekennzeichnet ist, dass die Spaltung in Gegenwart von sauren Molekularsieben, die gegebenenfalls durch eine Behandlung mit Wasserstoff aktiviert worden sind, durchgeführt wird.

Als für das erfindungsgemässe Verfahren verwendbare Molekularsiebe seien beispielsweise Zeolithe, saure Aluminiumoxide und H-Mordenite genannt. Als Zeolithe seien hierbei wasserhaltige Gerüstsilikate der allgemeinen Formel $x[(M', M''_{0,5})AlO_2] \cdot y\,SiO_2 \cdot zH_2O$ mit M' = Li, Na, K usw. und M'' = Mg, Ca, Sr, Ba usw. verstanden [Fortschr. Mineral 42, 50 (1965)]. Als H-Mordenite seien weiterhin orthorhombische Gerüstsilikate der Formel $Na_8[(AlO_2)_8 \cdot (SiO_2)_{40}] \cdot 24H_2O$ verstanden, deren Na-Atome sukzessive gegen H-Atome ausgetauscht sein können. [Am. Mineral, 39, 819 (1954)].

Bevorzugt werden H-Mordenite eingesetzt. Diese erfindungsgemäss einsetzbaren Molekularsiebe liegen in ihrer sauren $H^+$-Form vor.

Die beschriebenen sauren Molekularsiebe können sowohl einzeln als auch als Gemenge von 2 oder mehr der genannten Molekularsiebe erfindungsgemäss eingesetzt werden. Für den Fall, dass die Selektivität des erfindungsgemäss als Katalysator eingesetzten Molekularsiebs oder des Gemenges aus mehreren Molekularsieben erhöht werden soll, was im allgemeinen von einer Absenkung der Aktivität und/oder einer Absenkung des Durchsatzes begleitet ist, kann es von Vorteil sein, das oder die eingesetzten Molekularsiebe mit beispielsweise 0,01 bis 80 Gew.-%, bezogen auf die Menge der Molekularsiebe, an Aluminiumoxid und/oder amorphen Alumosilikaten zu mischen und das dabei entstandene Gemenge als Katalysator einzusetzen. Für eine spezielle Aufgabe im Rahmen der vorliegenden Erfindung kann die Notwendigkeit und gegebenenfalls der Umfang einer solchen Abmischung durch einfache Vorversuche ermittelt werden.

Die katalytisch wirksamen sauren Molekularsiebe für das erfindungsgemässe Verfahren besitzen ein effektives Porenvolumen von beispielsweise 0,05 bis 0,6, bevorzugt 0,12 bis 0,3 ml/g. Sie besitzen weiterhin einen effektiven Porendurchmesser von beispielsweise 3 bis 15, bevorzugt 7 bis 12, besonders bevorzugt 8 bis 11 Å. Die spezifische Oberfläche beträgt beispielsweise 100 bis 700, bevorzugt 300 bis 550 $m^2/g$.

Das erfindungsgemässe Verfahren wird beispielsweise bei einer Temperatur von 100 bis 300°C, bevorzugt 110 bis 210°C, besonders bevorzugt 120 bis 180°C, durchgeführt. Die Reaktion kann sowohl in der Gasphase als auch in der Flüssigphase bei einem Druck von beispielsweise 0,1 bis 50 bar, vorzugsweise 1 bis 20 bar, durchgeführt werden.

Für die Kontaktbelastung wird eine WHSV (weight-hourly-space-velocity) im Bereich von 0,5 bis 10 g Substrat/g Katalysator/Stunde gewählt.

Es wurde zusätzlich gefunden, dass der Katalysator für das erfindungsgemässe Verfahren

durch eine Behandlung mit Wasserstoff vor seinem katalytischen Einsatz eine Aktivitätssteigerung erfährt. Diese aktivitätssteigernde Wasserstoff-Behandlung des aus einem oder mehreren der oben beschriebenen Molekularsiebe und gegebenenfalls den beschriebenen Abmischungskomponenten bestehenden Katalysators kann sowohl vor seinem erstmaligen Einsatz als auch vor einem Wiedereinsatz eines bereits gebrauchten Katalysators erfolgen. Diese aktivitätssteigernde Wasserstoff-Behandlung bei einem bereits gebrauchten Katalysator kann sowohl dann vorgenommen werden, wenn der Katalysator Ermüdungserscheinungen zeigt, als auch als vorbeugende Massnahme, bevor Ermüdungserscheinungen am Katalysator erkennbar sind. Diese Wasserstoff-Behandlung kann beispielsweise mit 50 bis 500 Litern Wasserstoff pro Liter Katalysator bei 100 bis 450°C und 1 bis 50 bar für 1 bis 60 Stunden durchgeführt werden. Diese Wasserstoff-Behandlung kann selbstverständlich in einem separaten, dafür vorbestimmten Reaktor durchgeführt werden, ebensogut aber auch in dem für die erfindungsgemässe Etherspaltung vorgesehenen Reaktor, sofern dieser für die Wasserstoff-Behandlung ausgerüstet ist. So kann beispielsweise noch nicht benutzter Katalysator in den für die Etherspaltung vorgesehenen Reaktor eingefüllt werden, mit Wasserstoff unter den beschriebenen Bedingungen behandelt werden, wonach der zur Behandlung benutzte Wasserstoff, beispielsweise durch Spülung mit Stickstoff, entfernt wird und durch Einspeisung des zu spaltenden Ethers und Einstellung der erfindungsgemässen Bedingungen der Katalysator seiner Benutzung zugeführt wird. Weiterhin kann ein bereits benutzter Katalysator ohne Entfernung aus dem Spaltreaktor dadurch der Wasserstoff-Behandlung zugeführt werden, dass, beispielsweise durch Stickstoff, der bislang eingespeiste zu spaltende Ether verdrängt wird und anschliessend durch Einspeisung der gewünschten Menge Wasserstoff und Einstellung der beschriebenen Parameter für die Wasserstoff-Behandlung der Katalysator aktiviert wird.

n-Alkylether von tertiären Olefinen für das erfindungsgemässe Verfahren sind beispielsweise solche der allgemeinen Formel

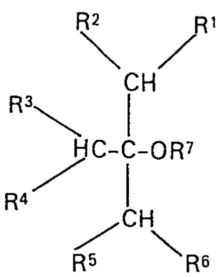

(I),

die erfindungsgemäss zu tertiären Olefinen der allgemeinen Formel

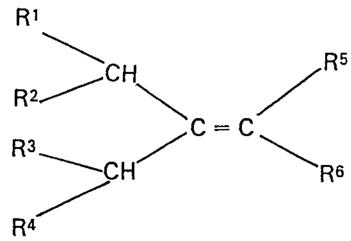

(II)

und n-Alkanolen der Formel

$R^7OH$ (III)

gespalten werden, worin

$R^1$ Wasserstoff oder geradkettiges oder verzweigtes Alkyl mit 1 bis 8, bevorzugt 1 bis 4, besonders bevorzugt 1 bis 2 C-Atomen,
$R^2$ bis $R^6$ Wasserstoff oder Alkyl mit 1 bis 4, bevorzugt 1 bis 2, besonders bevorzugt mit 1 C-Atomen und
$R^7$ geradkettiges oder verzweigtes Alkyl mit 1 bis 6, bevorzugt mit 1 bis 4, besonders bevorzugt 1 bis 2 C-Atomen, bedeuten.

Als Einsatzprodukte der Formel (I) für das erfindungsgemässe Verfahren seien beispielsweise folgende Ether genant: Methyl-tert.-butylether, Ethyl-tert.-butylether, Propyl-tert.-butylether, n-Butyl-tert.-butylether, n-Amyl-tert.-butylether, Methyl-tert.-amylether, Ethyl-tert.-amylether, Propyl-tert.-amylether, n-Butyltert.-amylether, n-Amyl-tert.-amylether, n-Hexyl-tert.-amylether, Methyl-tert.-hexylether und Ethyl-tert.-hexylether.

Die erfindungsgemäss erhältlichen Produkte sind neben den jeweiligen n-Alkanolen die tertiären Olefine, wie Isobuten, Isoamylen, Isohexen u.a.

Die Reingewinnung der erfindungsgemäss herstellbaren tertiären Olefine erfolgt in einer dem Spaltreaktor nachgeschalteten Destillationsstufe, wobei die gewünschten Olefine als Kopfprodukt in einer Reinheit von über 96 Gew.-% entnommen werden und ein Sumpfprodukt anfällt, das mit einer Zusammensetzung von etwa 90 bis 99,9 Gew.-% Alkanol, 0,1 bis 10 Gew.-% des eingesetzten Ethers und geringen Wassermengen in die Veretherung der tertiären Olefine zurückgeführt werden kann.

Das erfindungsgemässe Verfahren benötigt im Gegensatz zum Schwefelsäureextraktions-Verfahren zur Herstellung tertiärer Olefine einen Katalysator ohne korrosive Eigenschaften, so dass als Reaktormaterial normale Kohlenstoffstähle verwendet werden können. Gegenüber den oben beschriebenen katalytischen Spaltverfahren zeichnet sich das erfindungsgemässe Verfahren durch eine höhere Aktivität des zu verwendenden Katalysators aus, wodurch die katalytische Spaltung bei tieferen Temperaturen durchgeführt werden kann. Diese tiefere Reaktionstemperatur setzt den Zwangsanteil an unerwünschtem Di-n-Alkylether stark herab, so dass über 90% des im Kreislauf geführten n-Alkanols wiedergewonnen werden können, wobei gleichzeitig infolge der hohen Aktivität des erfindungsgemäss eingesetzten Katalysators hohe Ausbeuten und hohe Durchsätze bei der Spaltung erzielt werden. Die Absenkung der Reaktionstemperatur bringt gleichzeitig eine energiesparende Reaktionsführung mit sich.

Beispiele

Als Alkylether-Spaltreaktor wurde ein temperierbarer Durchlaufreaktor eingesetzt. Bei einem vorgegebenen lichten Reaktordurchmesser von 25 mm wurde die Katalysatorbetthöhe so gewählt, dass die Kontaktfüllung 100 g betrug. Zur Temperaturkontrolle war der Reaktor mit mehreren Temperaturmessungen im Abstand von 100 mm ausgerüstet. Der Reaktordruck wurde über eine Druckhaltung geregelt. Die Zudosierung des Substrats erfolgte über eine Membrankolbenpumpe. Die Zusammensetzung des am Reaktorausgang erhaltenen Reaktionsprodukts wurde gaschromatographisch untersucht. Der Produktstrom wurde in einer nachgeschalteten Destillationskolonne aufgearbeitet. Die gewünschten iso-Olefine wurden dabei als Kopfprodukt in einer Reinheit über 96 Gew.-% gewonnen.

Folgende Abkürzungen finden Verwendung: TAME = tert.-Amyl-methylether; MB = Methylbuten; DME = Dimethylether; WHSV = weight-hourly-space-velocity; GC = Gaschromatographie; MG = Molgewicht.

In den Beispielen 1–10 wurde die Temperaturabhängigkeit der Ether-Spaltung, in Verbindung mit der Dimethylether-Bildung untersucht.

Katalysator: 100 g $H^+$-Mordenit
Einsatzprodukt: 100 g TAME/h (GC 99,9 Gew.-%) (MG 102,2)

Reaktionsdruck 10 bar; Reaktionszeit 6 h;

$$WHSV = 1 \left( \frac{\text{g Substrat}}{\text{g Kontakt.h}} \right)$$

| Beispiel (Nr.) | | 1 | 2 | 3 | 4 | 5 |
|---|---|---|---|---|---|---|
| Temperatur (°C) | | 120 | 130 | 140 | 160 | 180 |
| TAME | Gew.-% | 32,3 | 24,3 | 18,7 | 12,7 | 2,1 |
| 2-MB-2 | Gew.-% | 34,0 | 37,2 | 41,0 | 44,0 | 53,4 |
| 2-MB-1 | Gew.-% | 12,5 | 14,8 | 14,8 | 15,9 | 13,8 |
| 3-MB-1 | Gew.-% | – | – | – | – | – |
| $CH_3OH$ | Gew.-% | 19,9 | 22,2 | 23,8 | 25,4 | 24,6 |
| DME | Gew.-% | 0,9 | 1,1 | 1,2 | 1,4 | 4,4 |
| $H_2O$ | Gew.-% | 0,4 | 0,4 | 0,5 | 0,6 | 1,7 |
| Umsatz TAME | % | 67,7 | 75,7 | 81,3 | 87,3 | 97,9 |
| Selektivität $CH_3OH$ | | 93,7 | 93,6 | 93,3 | 92,7 | 80,1 |
| Ausbeute $CH_3OH$ | % | 63,5 | 70,8 | 75,9 | 81,0 | 78,4 |

| Beispiel (Nr.) | | 6 | 7 | 8 | 9 | 10 |
|---|---|---|---|---|---|---|
| Temperatur (°C) | | 200 | 220 | 250 | 280 | 300 |
| TAME | Gew.-% | 1,2 | 0,9 | 0,4 | 0,2 | 0,2 |
| 2-MB-2 | Gew.-% | 51,9 | 53,2 | 53,5 | 52,9 | 53,4 |
| 2-MB-1 | Gew.-% | 15,9 | 14,8 | 14,8 | 15,4 | 14,8 |
| 3-MB-1 | Gew.-% | 0,1 | 0,1 | 0,1 | 0,2 | 0,3 |
| $CH_3OH$ | Gew.-% | 24,6 | 24,0 | 23,3 | 18,8 | 17,5 |
| DME | Gew.-% | 4,5 | 5,0 | 5,7 | 9,0 | 9,9 |
| $H_2O$ | Gew.-% | 1,8 | 2,0 | 2,2 | 3,5 | 3,9 |
| Umsatz TAME | % | 98,8 | 99,1 | 99,6 | 99,8 | 99,8 |
| Ausbeute $CH_3O$ | % | 79,6 | 77,4 | 74,3 | 59,9 | 55,9 |
| Selektivität $CH_3OH$ | | 78,4 | 76,5 | 74,3 | 59,9 | 55,8 |

In den Beispielen 11–13 wurde die Vorbehandlung des eingesetzten $H^+$-Mordenit mit $H_2$ untersucht. Daten der Vorbehandlung:

Katalysator: 100 g $H^+$-Mordenit
T-Vorbeh.: 190°C
p-Vorbeh.: 27 bar
t-Vorbeh.: 24 h
$H_2$: 40 l/h

Nach dieser Vorbehandlung wurde der $H_2$-Druck entspannt und mit $N_2$ der Reaktionsdruck von 10 bar eingestellt.

Als Reaktionsbedingungen wurde festgelegt:

p-Reaktion = 10 bar;
T-Reaktion = 140°C;
t-Reaktion = 24 h;

$$WHSV = 1 \left( \frac{\text{g Substrat}}{\text{g Kontakt.h}} \right)$$

Einsatzprodukt 100 g TAME/h (GC 99,9 Gew.-%).

| Beispiel (Nr.) | | 11 | 12 | 13 |
|---|---|---|---|---|
| $H_2$-Beh. | | (–) | (+) | (+) |
| TAME | Gew.-% | 18,7 | 3,8 | 4,4, |
| 2-MB-2 | Gew.-% | 41,0 | 49,2 | 48,3 |
| 2-MB-1 | Gew.-% | 14,8 | 16,8 | 17,3 |
| 3-MB-1 | Gew.-% | – | – | – |
| $CH_3OH$ | Gew.-% | 23,8 | 28,0 | 27,4 |
| DME | Gew.-% | 1,2 | 1,6 | 1,9 |
| $H_2O$ | Gew.-% | 0,5 | 0,6 | 0,7 |
| Umsatz TAME | % | 81,3 | 96,2 | 95,6 |
| Selektivität $CH_3OH$ | % | 93,3 | 92,7 | 91,3 |
| Ausbeute $CH_3OH$ | % | 75,9 | 98,3 | 87,1 |

+ Regeneration eines bereits gebrauchten Katalysators

In den Beispielen 14–18 wurde die Abhängigkeit der Ether-Spaltung von der Kontaktbelastung untersucht. Der eingesetzte $H^+$-Mordenit wurde analog den Beispielen 12 und 13 mit $H_2$ vorbehandelt.

Katalysator: 100 g $H^+$-Mordenit ($H_2$-aktiviert)
Einsatzprodukt: TAME (99,9 Gew.-%)
Reaktionsbedingungen: p-Reaktion = 10 bar;
T-Reaktion = 140°C;
t-Reaktion = 6 h
Kontaktbelastung: WHSV $\left(\frac{\text{g Substrat}}{\text{g Kontakt.h}}\right)$ 0,5–3,0

| Beispiel (Nr.) | | 14 | 15 | 16 | 17 | 18 |
|---|---|---|---|---|---|---|
| WHSV | | 0,5 | 1,0 | 1,5 | 2,0 | 3,0 |
| TAME | Gew.-% | 13,4 | 3,8 | 3,6 | 4,0 | 5,2 |
| 2-MB-2 | Gew.-% | 44,0 | 49,2 | 51,4 | 52,1 | 54,3 |
| 2-MB-1 | Gew.-% | 15,2 | 16,8 | 14,4 | 13,8 | 10,8 |
| 3-MB-1 | Gew.-% | – | – | – | – | – |
| $CH_3OH$ | Gew.-% | 23,5 | 28,0 | 28,0 | 28,2 | 28,2 |
| DME | Gew.-% | 2,8 | 1,6 | 1,6 | 1,4 | 0,7 |
| $H_2O$ | Gew.-% | 1,1 | 0,6 | 0,6 | 0,5 | 0,2 |
| Umsatz TAME | % | 86,3 | 96,2 | 96,4 | 96,0 | 94,8 |
| Selektivität $CH_3OH$ | % | 85,8 | 92,7 | 92,7 | 93,7 | 97,0 |
| Ausbeute $CH_3OH$ | % | 75,0 | 89,3 | 89,3 | 89,9 | 91,8 |

In den Beispielen 19–20 wurden verschiedene Alkyl-Ether auf ihre Spaltbarkeit untersucht.

Die Reaktionsbedingungen wurden analog zu den TAME-Spaltversuchen übernommen.

Eingesetzt wurde:
MTBE (Methyl-tert.-butyl-Ether), MG 88,2
TAEE (tert.-Amyl-ethyl-Ether), MG 116,2

Im Falle des MTBE erhält man als Spaltprodukt iso-Buten und Methanol

Im Falle des TAEE erhält man als Spaltprodukt iso-Amylene und Ethanol

Beispiel 19:
Katalysator: 100 g $H^+$-Mordenit ($H_2$-aktiviert)
Einsatzprodukt: MTBE (99,9 Gew.-%)
Reaktionsbedingungen: T-Reaktion = 140°C
p-Reaktion = 10 bar
t-Reaktion = 6 h
Kontaktbelastung:

$$WHSV = 1 \left(\frac{\text{g Substrat}}{\text{g Katalysator.h}}\right)$$

| | | |
|---|---|---|
| MTBE | Gew.-% | 9,2 |
| $i\text{-}C_4$ | Gew.-% | 57,8 |
| $CH_3OH$ | Gew.-% | 30,5 |
| DME | Gew.-% | 1,8 |
| $H_2O$ | Gew.-% | 0,7 |
| Umsatz MTBE | % | 90,8 |
| Selektivität $CH_3OH$ | % | 92,4 |
| Ausbeute $CH_3OH$ | % | 83,9 |

Beispiel 20:
Katalysator: 100 g $H^+$-Mordenit
Einsatzprodukt: TAEE (GC 99,9 Gew.-%)
Reaktionsbedingungen: T-Reaktion = 140°C
p-Reaktion = 10 bar
t-Reaktion = 6 h
Kontaktbelastung:

$$WHSV = 1 \left(\frac{\text{g Substrat}}{\text{g Katalysator.h}}\right)$$

| | | |
|---|---|---|
| TAEE | Gew.-% | 5,1 |
| 2-MB-2 | Gew.-% | 42,9 |
| 2-MB-1 | Gew.-% | 14,3 |
| 3-MB-1 | Gew.-% | - |
| $C_2H_5OH$ | Gew.-% | 34,1 |
| DEE | Gew.-% | 2,9 |
| $H_2O$ | Gew.-% | 0,7 |
| Umsatz TAEE | % | 94,9 |
| Selektivität $C_2H_5OH$ | % | 90,5 |
| Ausbeute $C_2H_5OH$ | % | 85,9 |

**Patentansprüche**

1. Verfahren zur Herstellung von tertiären Olefinen durch katalytische Spaltung ihrer n-Alkylether in Gegenwart saurer Katalysatoren bei einer Temperatur von 100–300°C und gleichzeitiger Wiedergewinnung der entsprechenden n-Alkanole, dadurch gekennzeichnet, dass die Spaltung in Gegenwart von sauren Molekularsieben, die gegebenenfalls durch eine Behandlung mit Wasserstoff aktiviert worden sind, durchgeführt wird.

2. Verfahren nach Anspruch 1, dadurch gekennzeichnet, dass H-Mordenit eingesetzt wird.

3. Verfahren nach Anspruch 1, dadurch gekennzeichnet, dass ein Gemenge von mindestens einem sauren Molekularsieb mit 0,01 bis 80 Gew.-%, bezogen auf die Menge des Molekularsiebs, an Aluminiumoxid und/oder Alumosilikat eingesetzt wird.

4. Verfahren nach Anspruch 1 bis 3, dadurch gekennzeichnet, das die sauren Molekularsiebe vor ihrem Einsatz oder ihrem wiederholten Einsatz mit 50 bis 500 l Wasserstoff pro Liter Molekularsieb bei 100 bis 450°C und 1 bis 50 bar für 1 bis 60 Stunden behandelt werden.

**Claims**

1. Process for the preparation of tertiary olefines by catalytic cleavage of their n-alkyl ethers in the presence of acid catalysts at a temperature of 100–300°C and with simultaneous recovery of the corresponding n-alkanols, characterised in that the cleavage is carried out in the presence of acidic molecular sieves which have been activated, if necessary, by a treatment with hydrogen.

2. Process according to Claim 1, characterised in that H-mordenite is used.

3. Process according to Claim 1, characterised in that a mixture of at least one acidic molecular sieve with 0.01 to 80% by weight, based on the amount of the molecular sieve, of aluminium oxide and/or aluminosilicate is employed.

4. Process according to Claim 1 to 3, characterised in that the acidic molecular sieves, prior to their use or their repeated use, are treated with 50 to 500 l of hydrogen per litre of molecular sieve at 100 to 450°C and 1 to 50 bar for 1 to 60 hours.

**Revendications**

1. Procédé de préparation d'oléfines tertiaires par dissociation catalytique de leurs éthers n-alkyliques en présence de catalyseurs acides à une température de 100–300°C, avec récupération simultanée des n-alcanols correspondants, caractérisé en ce qu'on effectue la dissociation en présence de tamis moléculaires acides qui ont été éventuellement activés par un traitement avec l'hydrogène.

2. Procédé selon la revendication 1, caractérisé en ce qu'on utilise la mordénite H.

3. Procédé selon la revendication 1, caractérisé en ce qu'on utilise un mélange d'au moins un tamis moléculaire acide avec 0,01 à 80% en poids (calculé sur la quantité du tamis moléculaire) d'oxyde d'aluminium et/ou d'aluminosilicate.

4. Procédé selon les revendications 1 à 3, caractérisé en ce que, avant leur utilisation ou leur réutilisation, on traite les tamis moléculaires acides avec 50 à 500 litres d'hydrogène par litre de tamis moléculaire à une température de 100 à 450°C et sous une pression de 1 à 50 bars pendant une période de 1 à 60 heures.